# EUROPÄISCHE PATENTSCHRIFT

(11) **EP 0 670 702 B1**
(45) Veröffentlichungstag und Bekanntmachung des Hinweises auf die Patenterteilung: **11.04.2007**
(21) Anmeldenummer: 94926078.0
(22) Anmeldetag: 20.09.1994
(51) Int. Cl.: A61F 2/44, A61F 2/46

(54) **IMPLANTAT FÜR DEN ZWISCHENWIRBELRAUM**
IMPLANT FOR THE INTERVERTEBRAL SPACE
IMPLANT POUR L'ESPACE INTERVERTEBRAL

(30) Priorität: 21.09.1993 BE 9300982
(43) Veröffentlichungstag der Anmeldung: 13.09.1995
(60) Teilanmeldung: 04007507.9
(73) Patentinhaber: Synthes GmbH, 4436 Oberdorf (CH)
(72) Erfinder: BECKERS, Louis, François, Charles, B-2820 Rijmenam (BE); SCHLÄPFER, Johannes, Fridolin, CH-8750 Glarus (CH)
(74) Vertreter: Lusuardi, Werther
(86) Internationale Anmeldenummer: PCT/CH1994/000184
(87) Internationale Veröffentlichungsnummer: WO 1995/008306

(56) Entgegenhaltungen:
- EP-A- 0 260 044
- EP-A- 0 307 241
- EP-A- 0 493 698
- WO-A-89/12431
- WO-A-90/00037
- WO-A-92/14423
- WO-A-93/01771
- US-A- 3 486 505
- US-A- 4 349 921
- US-A- 4 772 287
- US-A- 5 092 893

## Beschreibung

Die Erfindung bezieht sich auf ein Implantat für den Zwischenwirbelraum gemäss dem Oberbegriff des Patentanspruchs 1. Solche Implantate sind hauptsächlich dazu bestimmt, Knochenbrücken an Wirbelkörpern zu fördern und welche nach der Resektion von Diskus bzw. Zwischenwirbelscheibe zwischen Wirbelkörper und Rückgrat angebracht werden.

Es ist bekannt, dass bei Beschädigung einer Zwischenwirbelscheibe diese entfernt und der entstandene Raum mit kortikospongiösem Knochen gefüllt werden kann.

Bei dieser Methode werden die Wirbelkörper zuerst weitmöglichst mit Hilfe von Spreizer auseinandergedehnt. Eine Spezialtechnik besteht darin, dass keilförmige Elemente - sogenannte Dilatatoren - zwischen die beiden Wirbelkörper eingeführt werden, um sie schrittweise auseinander zu dehnen. Dabei wird abwechselnd links und rechts jeweils ein Dilatator mit einem 1 mm grösseren Durchmesser von posterior angebracht. Nachdem die grösstmögliche Dehnung erreicht ist, werden die Dilatatoren durch den obengenannten kortiko-spongiösen Knochen ersetzt.

Diese bekannte Technik hat den Nachteil, dass der Knochen schwierig zu handhaben und in die richtige Position zu bringen ist, wobei Korrekturen nahezu ausgeschlossen sind. Ein weiterer Nachteil dieser Technik besteht darin, dass im Zwischenwirbelraum eine rechteckige oder zylinderförmige Aussparung ausgestochen und/oder ausgefräst werden muss, um die Knochenpfropfen zwischen die ursprünglich konkaven Seiten der angrenzenden Wirbelkörper bringen zu können, was umständlich ist und zusätzlich zur Beschädigung der Wirbelkörper führt.

Dokument EP-0 307 241 offenbart ein Implantat für den Zwischenwirbelraum gemäß dem Oberbegriff von Anspruch 1.

Hier will die Erfindung Abhilfe schaffen. Der Erfindung liegt die Aufgabe zugrunde, ein Implantat für den Zwischenwirbelraum zu schaffen, welches aufgrund seiner spezifischen Form und der Einbringungsart eine äusserst stabile Verklemmung zwischen den Wirbelkörpern ermöglicht, ohne dass dabei die Oberfläche der knöchernen Deckplatte der Wirbelkörper beschädigt wird.
Eine weitere Aufgabe der Erfindung liegt in der Schaffung eines Implantates für den Zwischenwirbelraum zu schaffen, welches ohne Verwendung von Dilatatoren eingebracht werden kann.

Die Erfindung löst die gestellte Aufgabe mit einem Implantat für den Zwischenwirbelraum, welches die Merkmale des Anspruchs 1 aufweist.
Weitere vorteilhafte Ausgestaltungen der Erfindung sind in den abhängigen Ansprüchen gekennzeichnet.

Da das erfindungsgemässe Implantat mit einer Vorrichtung zur Ergreifung durch ein Werkzeug versehen ist, kann relativ mühelos eine externe Kraft darauf ausgeübt werden, die es ermöglicht, das Implantat nach der Anbringung zu bewegen oder es eventuell wieder herauszunehmen.

Die Vorrichtungen zur Ergreifung durch ein Werkzeug können als Ansatzpunkte derart gestaltet sein, dass eine Rotationskraft und/oder eine axiale Kraft und/oder eine seitliche Kraft auf das Implantat ausgeübt werden kann.

Bei einer vorteilhaften Ausführungsform sind diese Ansatzpunkte zumindest so gestaltet, dass sie die Ausübung einer Rotationskraft auf das Implantat ermöglichen, wobei das Implantat dabei unterschiedliche Querschnittslängen aufweisen muss, damit es durch die Drehung des Implantats mehr oder weniger eingeklemmt wird oder selbst in einer Position völlig lose sitzen und somit mühelos zwischen die Wirbelkörper gebracht werden kann und in einer anderen Position die erforderliche Einklemmung aufweist.

Bei einer anderen Ausführungsform weist der Körper des Implantats in einer Ebene ein linsenförmig zugeschnittenes Profil auf, das grösstenteils mit der bikonkaven Form der sagittalen Schnittfläche des Zwischenwirbelraums übereinstimmt, wobei derselbe Körper in der anderen Ebene hauptsächlich parallele, flache oder nur leicht gebogene Seiten und ein abgerundetes Ende aufweist, damit er in den Zwischenwirbelraum gedrückt werden kann, ohne eine Aussparung in den Wirbelkörper stechen zu müssen und ohne die Umrandung des Wirbelkörpers zu beschädigen.

Das Implantat ist vorzugsweise hohl, damit es mit Knochenmaterial gefüllt werden kann.

Um die Erfindung besser zu verdeutlichen, werden nachstehend einige Beispiele vorteilhafter Ausführungsformen - auf die sich die Erfindung jedoch nicht beschränkt - mit Verweisen nach den entsprechenden Zeichnungen beschrieben.

Es zeigen:
Fig. 1 eine schematische Darstellung zweier Wirbelkörper, die mit zwei Dilatatoren auseinandergedehnt sind;
Fig. 2 einen Querschnitt entlang der Linie II-II in Fig. 1, wobei ein Dilatator durch einen kleinen Knochenquader ersetzt ist;
Fig. 3 eine perspektivische Darstellung eines erfindungsgemässen Implantats mit einem dazu verwendbaren Werkzeug;
Fig. 4 eine Aufsicht in Richtung des Pfeiles F4 der Fig. 3;
Fig. 5 eine Aufsicht in Richtung des Pfeiles F5 der Fig. 3;
Fig. 6 einen Querschnitt entlang der Linie VI-VI in Fig. 4;
Fig. 7 eine schematische Darstellung des Implantats nach Fig. 3 nach erfolgter Einführung zwischen zwei Wirbelkörpern;
Fig. 8 eine schematische Darstellung des Implantats nach Fig. 3 nach erfolgter Einführung zwischen zwei Wirbelkörpern und Rotation um 90°;
Fig. 9 eine schematische Darstellung einer weiteren Ausführungsform der Erfindung mit einem dazu verwendbaren Werkzeug;
Fig. 10 einen Querschnitt durch ein erfindungsgemässes Implantat mit einseitiger Abrundung;
Fig. 11 einen Querschnitt durch ein erfindungsgemässes Implantat mit doppelseitiger Abrundung über die Diagonale;
Fig. 12 einen Querschnitt durch eine paarige Anordnung zweier spiegelsymmetrischer erfindungsgemässer Implantate;
Fig. 13 eine schematische Darstellung paarig angeordneter, spiegelsymmetrischer erfindungsgemässer Implantate mit deren Hilfe der Bandscheibenraum distrahiert werden kann;
Fig. 14 eine schematische Darstellung von zwei flach im Bandscheibenraum liegenden Implantaten, die über ein drittes Implantat anterior verbunden sind, vor und nach der Rotation in die Konkavität der Deckplatten der angrenzenden Wirbelkörper;
Fig. 15 eine perspektivische Darstellung eines erfindungsgemässen Implantates mit einem Längsschnitt zur Aufnahme von spongiösen Knochenmaterial oder osteokonduktives bzw. osteoinduktives Material und einer Querperforation der Wände für das Knochenwachstum;
Fig. 16 eine perspektivische Darstellung eines erfindungsgemässen Implantates mit längsstrukturierten Kontaktflächen zwischen Implantat und Knochen, wobei die Längsstrukturierung derart gestaltet ist, dass das Rotieren des Implantates in die Konkavität der Deckplatten nur in einer Richtung möglich ist;
Fig. 17 eine perspektivische Darstellung eines erfindungsgemässen Implantates mit querstrukturierten Kontaktflächen zwischen Implantat und Knochen, wobei die Querstrukturierung derart gestaltet ist, dass die eine Strukturierung eine Translation in anteriorer und die andere eine Translation in posteriorer Richtung verhindert. Die Verhinderung der Translation in anteriorer Richtung führt zu einer Entlastung des verbliebenen Annulus, der nach jüngster Forschung innerviert ist und damit auf anterioren Druck mit Schmerzsignalen reagieren könnte.

Die Erfindung und Weiterbildungen der Erfindung werden im folgenden anhand der teilweise schematischen Darstellungen mehrerer Ausführungsbeispiele noch näher erläutert.

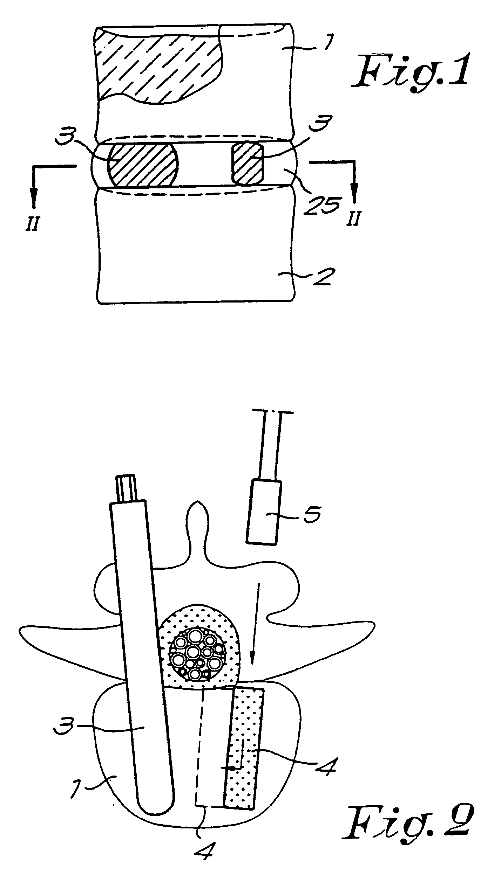

Anhand der Figuren 1 und 2 wird vorerst die bekannte Technik beschrieben.
Wenn eine Zwischenwirbelscheibe entnommen wird, werden, wie in Fig. 1 dargestellt, die zwei angrenzenden Wirbelkörper 1 und 2 soweit als möglich auseinandergedehnt, um die Dilatatoren 3 anbringen zu können. Nachdem die Wirbelkörper 1 und 2 sich im gewünschten Abstand befinden, werden die Dilatatoren 3, wie in Fig. 2 dargestellt, durch die Knochenpfropfen 4 ersetzt, die nach dem Ausstechen einer Aussparung in den Wirbelkörpern 1 und 2 mit einem Andruckelement 5 zwischen die Wirbelkörper gepfropft werden. Es ist ersichtlich, dass diese Technik die in der Beschreibungseinleitung genannten Nachteile aufweist.

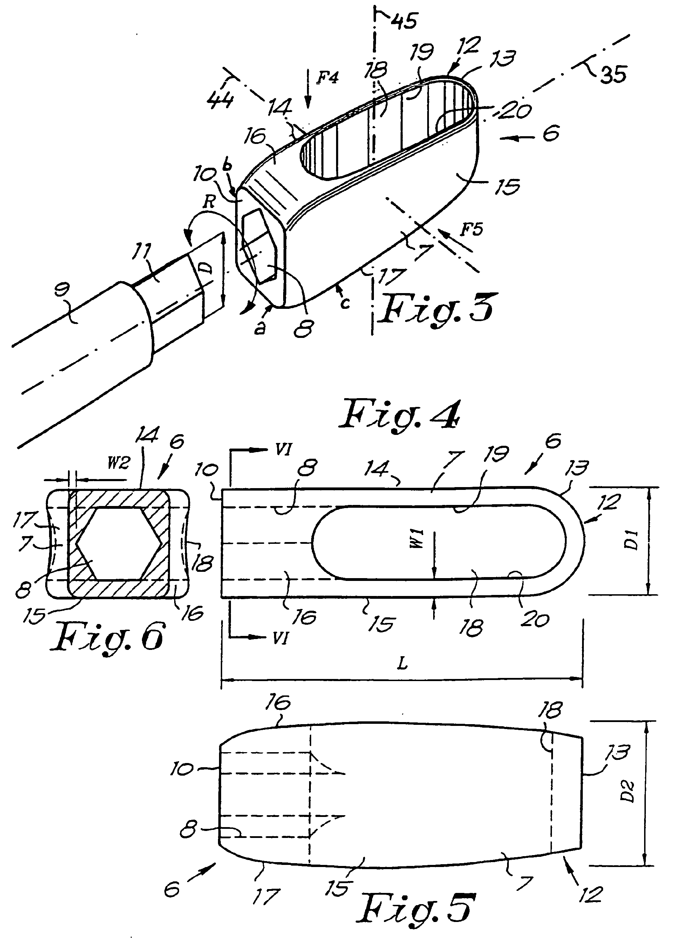

Das in den Fig. 3 - 6 dargestellte, erfindungsgemässe Implantat, überwindet nun diese Nachteile und erlaubt, dass es schnell eingebracht und zusätzlich, falls erforderlich, unter Kraftanwendung zwischen zwei Wirbelkörpern geklemmt werden kann. Das Implantat 6 besteht im wesentlichen aus einem Körper 7 mit einer Vorrichtung 8 zur Ergreifung durch ein Werkzeug 9.
Die Vorrichtungen 8 zur Ergreifung durch eine Werkzeug 9 sind so gestaltet, dass eine Rotationskraft, eine axiale Kraft und/oder eine seitliche Kraft auf das Implantat 6 ausgeübt werden kann, vorzugsweise in allen Richtungen.

Vorteilhafterweise sind, wie in den Fig. 3 - 6 dargestellt, die Vorrichtungen 8 derart gestaltet, dass darauf mindestens eine Rotationskraft R ausgeübt werden kann und in Verbindung damit das Implantat so gestaltet ist, dass es verschiedene Durchmesser oder Querschnittslängen aufweist, damit durch Drehung an den genannten Vorrichtungen 8 der Körper 7 des Implantats 6 mit grossen oder kleinen Abständen zwischen die Wirbelkörper 1 und 2 gebracht werden kann.

Die Vorrichtungen 8 bestehen bei der Ausführungsform nach den Fig. 3 - 6 aus einer in der Innenseite des Körpers 7 angebrachten Aussparung am hinteren axialen Ende 10 des Implantats 6. Die Aussparung ermöglicht es, ein Werkzeug 9 einzuführen. Wie dargestellt, kann die Aussparung aus einer axialen mehrkantigen, z.B. sechskantigen Öffnung bestehen, wobei hierbei ein Werkzeug 9 verwendet werden muss, das mit einem sechskantigen Ende 11 in der Form eines Imbusschlüssels versehen ist.

Die Verwendung einer in der Innenseite angebrachten Öffnung für das Einführen eines Werkzeugs 9, also die vorgenannte Aussparung, bietet den Vorteil, dass am Implantat 6 keine störenden Elemente hervorstehen.

Der Körper 7 weist vorzugsweise eine besondere Form mit einem oder mehreren der hiernach aufgeführten Kennzeichen auf:
- das vordere axiale Ende 12 des Körpers 7 sollte abgerundet oder keilförmig ausgebildet sein, da dies die Einführung in den Zwischenwirbelraum 25 erleichtert;
- die Abrundung 13 am vorderen axialen Ende 12 des Körpers 7 verläuft vorzugsweise nur entlang einem Querschnitt parallel zum kleinsten Durchmesser D1 - siehe Fig. 4 - und nicht nach dem dazu im rechten Winkel stehenden Querschnitt, wie in Fig. 5 dargestellt.
- die Seiten 14 und 15, durch die der kleinste Durchmesser verläuft, sind mit Ausnahme der Abrundung 13 vorzugsweise parallel und flach;
- bei Seitenansicht weist der Körper 7 wie in Fig. 5 dargestellt ein abgekantetes, linsenförmiges Profil auf, also ein Profil, das mit der natürlichen bikonkaven Form übereinstimmt, die ein Zwischenwirbelraum in der sagittalen Schnittfläche aufweist. Die Übergänge zwischen den Seiten 14 und 15 und die Seiten 16 und 17 sind abgerundet;
- die Seiten 16 und 17 sind vorzugsweise entlang einem Querschnitt mindestens teilweise und besser vollkommen flach; die Tatsache, dass die Seiten 17 und 18 in Querrichtung mindestens teilweise flach sind, bietet den Vorteil, dass sie in eingeklemmtem Zustand Kippstabilität bieten;
- der Körper 7 weist eine oder mehrere Öffnungen oder Aussparungen für die Füllung mit Pfropfmaterial auf; gemäss den Fig. 3 bis 6 wird eine durchgehende, sich von Seite 16 bis Seite 17 erstreckende Öffnung 18 bevorzugt; die Öffnung 18 besteht vorzugsweise aus einem länglichen Schlitz mit den parallelen Wänden 19 und 20; die vorgenannte Aussparung 8 kann sich dabei auf Wunsch bis in die Öffnung 18 erstrecken.
- Das Implantat wird vorzugsweise aus Titan oder einer für Implantate geeigneten Titanlegierung gefertigt.
- Die Öffnung 18 oder der Schlitz im Körper 7 des Implantats der Fig. 3 kann angebracht werden, wenn mehrere vertikale Bohrungen im Körper 7 gemacht und die Zwischenwände danach weggefräst werden;
- vorzugsweise weist das Implantat 6, und genauer gesagt der Körper 7 eine Länge L von ungefähr 22 mm auf und wird ausgehöhlt, bis ungefähr auf eine Wanddicke W1 von 1,5 mm bestehen bleibt. Das hintere axiale Ende 10 mit der Vorrichtung 8 weist vorzugsweise einen Mindestdurchmesser von 6 mm auf; um zu erreichen, dass die Mindestwanddicke W2 an der Stelle der Vorrichtung 8 und die Dicke D des Werkzeugs grösstmöglich ist, wird die vorgenannte Aussparung so angebracht, dass die Richtung ihres grössten Durchmessers mit der des grössten Durchmessers des Körpers 7 übereinstimmt.

Anhand der Fig. 7 und 8 wird nun nachstehend die Verwendung und das Anbringen des Implantats 6 zwischen zwei Wirbelkörpern 1 und 2 beschrieben.
In Fig. 7 wird dargestellt, wie das Implantat 6 auf dem Ende eines einem Schlüssel ähnelnden entsprechenden Werkzeugs 9 zwischen den beiden Wirbelkörpern 1 und 2 angebracht werden kann. Das Implantat 6 wird dabei mit dem kleinsten Durchmesser D1 zwischen die zueinander gerichteten Seiten 22 und 23 der Wirbelkörper 1 und 2 eingeführt. Dabei ist es bereits mit Knochenpfropfen 24 gefüllt. Um das Implantat 6 danach passend oder klemmend zwischen den Wirbelkörpern 1 und 2 anzubringen, wird der Schlüssel 21 des Werkzeugs 9 um 90° gedreht, damit, nach Entfernung des Werkzeugs 9, ein wie in Fig. 8 dargestellter Zustand erreicht wird. Da die Knochenpfropfen 24 an die Wirbelkörper 1 und 2 anschliessen, kann das Implantat 6 durch Verwachsung der Knochenpfropfen 24 festen Halt erreichen.

Das Implantat 6 kann ohne besondere Hilfsmittel eingebracht werden, der Vorgang kann jedoch vereinfacht werden, wenn die Wirbel vorher mittels ovaler Dilatatoren an der linken und rechten Seite auseinandergedehnt und so lange in dieser Position beibehalten werden, bis an der anderen Seite ein Implantat 6 eingeklemmt ist. Da die Anwesenheit des Implantats 6 dann wiederum verhindert, dass sich die Wirbelflächen erneut zusammenschieben, kann jetzt der letzte Dilatator entfernt und eventuell durch ein zweites Implantat 6 ersetzt werden. Normalerweise müssen zwei Implantate 6 angebracht werden.

Aus den Fig. 7 und 8 wird deutlich ersichtlich, dass bei der Verwendung eines drehbaren Implantats 6 mit unterschiedlichen Durchmessern D1 und D2 dieses frei und ohne viel Mühe zwischen die Wirbelkörper 1 und 2 eingeführt werden kann und es andererseits durch Drehung in perfekten Halt zwischen die Wirbelkörper gebracht werden kann. Daher ist es auch nicht erforderlich, den Zwischenwirbelraum 25 für den Erhalt eines rechteckigen oder zylinderförmigen Raumes auszustechen oder auszufräsen.

Da der Körper 7 des Implantats 6 unterschiedliche Durchmesser D1 und D2 aufweist, ist er einfach aus dem Zwischenwirbelraum 25 zu entfernen. Es ist eindeutig, dass das Implantat 6 nach dem Einklemmen erneut gelöst werden kann, wenn es in entgegengesetzte Richtung gedreht wird, bis sich der kleinste Durchmesser D1 zwischen den Wirbelkörpern 1 und 2 befindet.

Wenn ein Implantat 6 verwendet wird, das einen Körper 7 mit einer Form aufweist, die mit der natürlichen bikonkaven Form des Zwischenwirbelraums 25 übereinstimmt, entsteht automatisch ein perfekter Anschluss zwischen den Seiten 22 und 23 der Wirbelkörper 1 und 2 und den Seiten 16 und 17 des mit Knochenpfropfen 24 verpfropften Implantats 6.
Die Technik des Drehens des Implantates 6 hat folgende Vorteile:
- Wenn die Deckplatten konkav gewölbt sind, dann bringt das Rotieren die Möglichkeit, das Implantat 6 derart zu gestalten, dass es in einer Dimension flach ist und in der anderen Dimension der Geometrie der Deckplatte entspricht. Die flache Dimension erleichtert das Einschieben von posterior; die gewölbte Fläche ergibt einen optimalen Kontakt mit den Deckplatten;
- wenn die Deckplatten flach sind, dann kann das Rotieren benutzt werden, um den Bandscheibenraum zu spreizen;
- eine Querverzahnung der Oberfläche des Implantates ist möglich, da das Implantat erst nach der Insertion gedreht wird.

Selbstverständlich kann das Implantat 6 in verschiedenen Formen verwirklicht werden. Anstelle einer Aussparung für einen Sechskantimbusschlüssel können auch andere Aussparungsformen verwendet werden, die z.B. aus vierkantigen, rechteckigen oder ovalen Öffnungen bestehen.

Obwohl die Vorrichtungen 8 zur Ergreifung durch ein Werkzeug 9 vorzugsweise in der Innenseite des Implantats 4 angebracht sind, ist dies nicht unbedingt erforderlich. Sie können auch aus einem vorstehenden Teil oder aus einer bestimmten Formgebung des hinteren axialen Endes 10 bestehen, so dass der vorstehende Teil oder das hintere axiale Ende 10 an einem geeigneten Werkzeug befestigt werden kann, um die erforderliche Kraft ausüben zu können.

Nach einer anderen Ausführungsform der Erfindung sind die Vorrichtungen 8 nicht ausschliesslich dafür vorgesehen, dass darauf eine Rotationskraft, sondern zugleich eine Axialkraft ausgeübt werden kann und zwar sowohl eine Druck-, wie eine Zug-Kraft, damit, falls erforderlich, das Implantat 6 bei der Anbringung zwischen den Wirbelkörpern 1 und 2 eingedrückt und bei eventueller erneuter Entnahme, bei Verklemmung, eine Zugkraft darauf ausgeübt werden kann. Damit ist es jederzeit möglich, das Implantat 6 während des Eingriffs erneut zu entfernen.

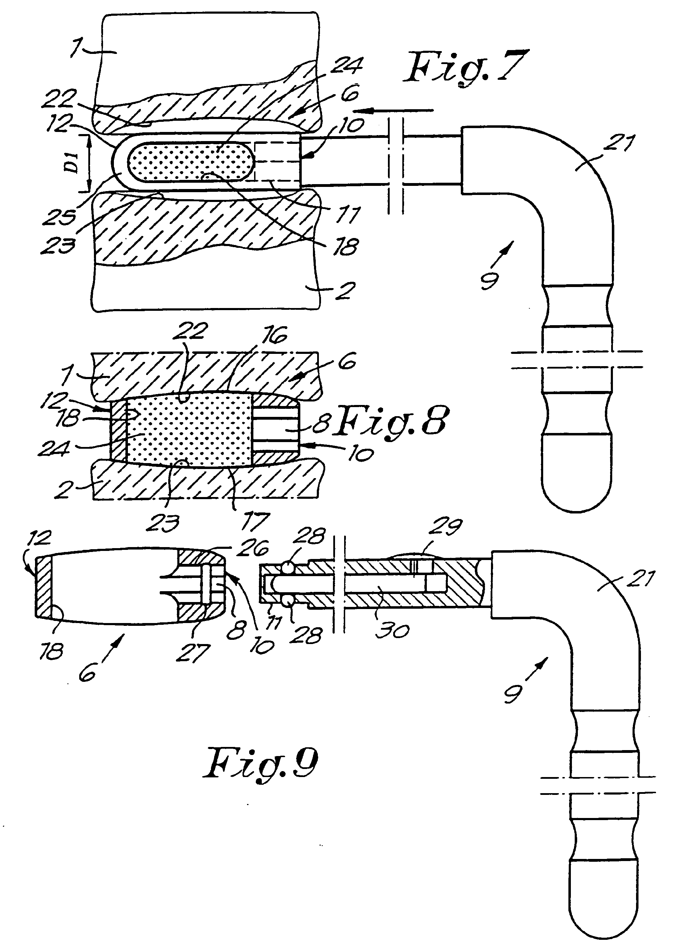

Eine derartige Ausführungsform ist anhand der Fig. 9 dargestellt. Die Vorrichtungen 8 verbinden dabei ein erstes Ansatzelement 26, welches die Ausübung einer Rotationskraft zulässt, mit einem zweiten Ansatzelement 27, welches die Ausübung einer axialen Druck- und Zugkraft auf das Implantat 6 ermöglicht und dazu mit einer Axialsperre versehen ist.

Das erste Ansatzelement 26 besteht aus einer wie in der Ausführungsform nach Fig. 3 dargestellten Aussparung. Das zweite Ansatzelement 27 besteht aus einer zusätzlichen Aussparung, z.B. in Form eines Schlitzes in der Wand der obengenannten sechskantigen Öffnung, in den die Sperrelemente 28 des betreffenden Werkzeugs 9 greifen können. Wie in Fig. 9 dargestellt, können die Sperrelemente 28 aus Kugeln oder ähnlichem bestehen, die, nachdem das sechskantige Ende 11 des Werkzeugs 9 in die sechskantige Aussparung eingeführt wurde, radial nach aussen gedrückt werden und in den obengenannten Schlitz greifen.

Das Werkzeug 9 kann dabei verschiedene Formen aufweisen und auf unterschiedliche Art bedient werden. Gemäss Fig. 9 wird dies mit einem mit einem Keil 30 verbundenen Umschaltgriff 29 bewerkstelligt, der wiederum die Sperrelemente 28 auseinanderdrückt oder löst.

Bei einer anderen Variante ist das Schlüsselende gespalten, der Aussendurchmesser kann durch Andrücken oder Anschrauben eines inneren Stifts vergrössert werden, damit der Schlüssel in der Öffnung des Implantats 6, in das er eingeführt wird, eingeklemmt werden kann.

Nach einer anderen Variante können auch am vorderen axialen Ende 12 mit der Abrundung 13 des Implantats 6 Ansatzmöglichkeiten für ein Werkzeug 9 vorgesehen werden. Diese Ansatzmöglichkeiten können von unterschiedlicher Art sein und sind vorzugsweise derart gestaltet, dass sie, gleich wie die Vorrichtungen 8, die Ausübung einer Rotationskraft, einer axialen Kraft und/oder einer seitlichen Kraft auf das Implantat 6 ermöglichen. Die Ansatzmöglichkeiten bestehen aus einer mehrkantigen, z.B. sechskantigen Öffnung, die die Anbringung eines Schlüssels mit entsprechendem Endstück ermöglicht, damit eine Torsionskraft auf das Implantat 6 ausgeübt werden kann, nachdem es nicht ausreichend fest angewachsen ist und auf abdominalem Weg entfernt werden muss. Diese Erfindung bezieht sich selbstverständlich auch auf Implantate 6, die an einem Ende mit Ansatzvorrichtung versehen sind, die eine Anbringung der Implantate auf abdominalem Weg ermöglichen.

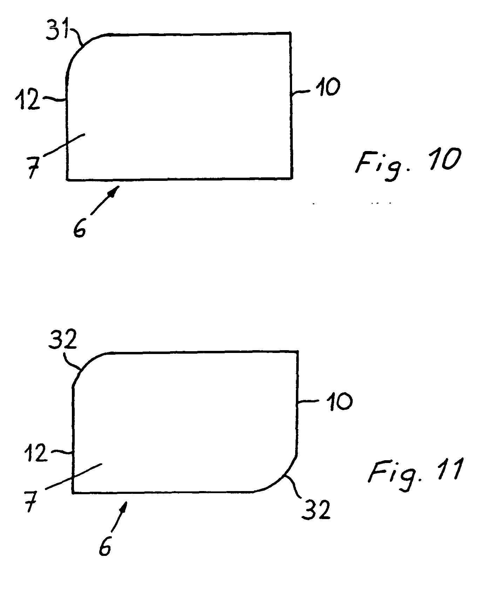

In den Fig. 10 und 11 sind erfindungsgemässe Implantate dargestellt, welche einen teilweise abgerundeten Querschnitt aufweisen.

Fig. 10 zeigt den Körper 7 eines Implantates 6, welcher an der oberen Kante des vorderen axialen Endes 12 eine Abrundung 31 aufweist. Der Radius der einseitigen Abrundung 31 ist derart bemessen ist, dass a) die Differenz zwischen der grösseren Seite des rechteckigen Querschnittes und der Diagonale über die abgerundete Kante kleiner als 3 mm, vorzugsweise 1 - 2 mm beträgt; und b) die kleinere Fläche um weniger als die Hälfte, vorzugsweise um weniger als ein Drittel reduziert ist, d.h. die tragende Fläche sollte mindestens 2/3 der Gesamtbreite des Implantates entsprechen.

Fig. 11 zeigt den Körper 7 eines Implantates 6 im Querschnitt, wobei das Implantat im Querschnitt über die Diagonale je eine Abrundung 32 aufweist. Die Radien der gegenseitigen Abrundungen 32 sind derart bemessen, dass a) die Differenz zwischen der grösseren Seite des Querschnittes und der Diagonale über die abgerundeten Kanten kleiner als 3 mm, vorzugsweise 0,5 - 1,0 mm beträgt und b) die kleinere Fläche des Implantates um weniger als die Hälfte, vorzugsweise kleiner als ein Drittel reduziert ist.

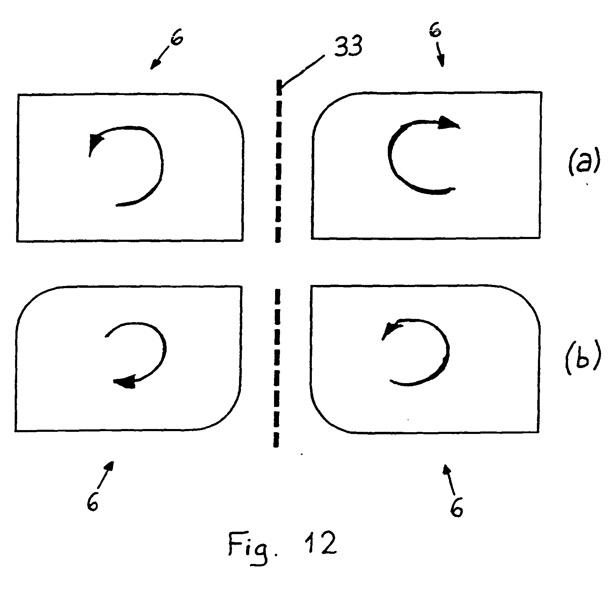

In Fig. 12 sind zwei bezüglich der Symmetrieachse 33 symmetrisch angeordnete Paare von Implantaten 6 dargestellt, wobei die beiden oberen Implantate 6 im Abschnitt (a) solche gemäss der Fig. 10 und die beiden unteren Implantate 6 im Abschnitt (b) solche gemäss der Fig. 11 darstellen.

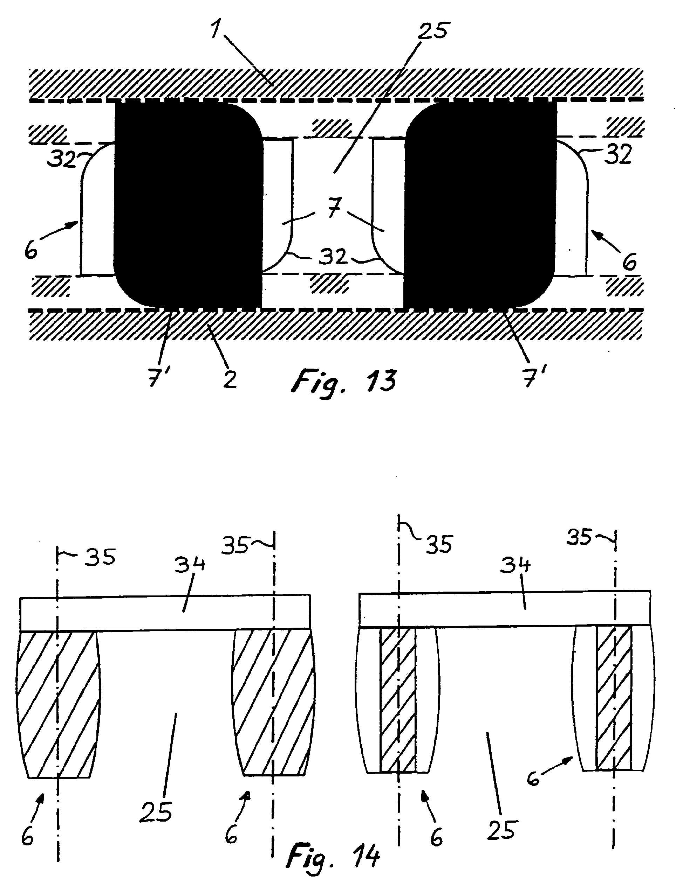

Beim Aufrichten (Rotieren) eines wie in Fig. 6 gezeigten Implantates 6 wird der Zwischenwirbelraum 25 um etwa 3-4 mm überdehnt, was zu einem Einbrechen der Deckplatten und permanenten Überdehnen des Bindegewebes führen kann. Wenn die Kanten nun eine Abrundung (31,32) aufweisen, wird die Überdehnung stark reduziert, damit ist aber auch die Stabilität der aufgerichteten Implantate reduziert oder die gepaarten Implantate sind spiegelsymmetrisch angeordnet um sich gegenseitig zu stabilisieren (siehe Fig. 13).
Die Aufrichtung mittels zweier Implantate 6, welche mit Abrundungen 31 oder 32 versehen sind führt bei geeignet gewählten Radien der Abrundungen 31 oder 32 zu einer Überdistrahierung des Zwischenwirbelraumes 25 von nur 1 mm; dafür sind aber die einzelnen aufgerichteten Implantate 6 nicht allzu stabil; sie können so leicht zurückkippen wie sie aufzurichten waren. In Fig. 13 schützen sich die beiden Implantate 6 durch die spiegelsymmetrische Geometrie gegenseitig vor dem Umkippen, da die Implantate 6 nur im Verbund und nicht einzeln umkippen können.

In Fig. 13 sind zwei spiegelsymmetrische angeordnete Implantate 6 gemäss Fig. 11 dargestellt. Die Abrundungen 32 der Körper 7 kommen dabei symmetrisch zueinander zu liegen. Die Körper 7 der Implantate 6 liegen nach deren Einführung horizontal zwischen den Wirbelkörper 1 und 2 und können dann mittels eines geeigneten Werkzeugs 9 um 90° in die schwarz gezeichnet Stellung 7' rotiert werden um den Zwischenwirbelraum 25 distrahieren zu können. Der rechteckige Querschnitt der Körper 7 ist derart beschaffen, dass nach der Rotation des Implantates 6 um 90° in die Konkavität der Deckplatten der angrenzenden Wirbelkörper 1,2 eine Distraktion des Zwischenwirbelraumes 25 zwischen 1 und 4 mm, vorzugsweise zwischen 2 - 3 mm, verbleibt.

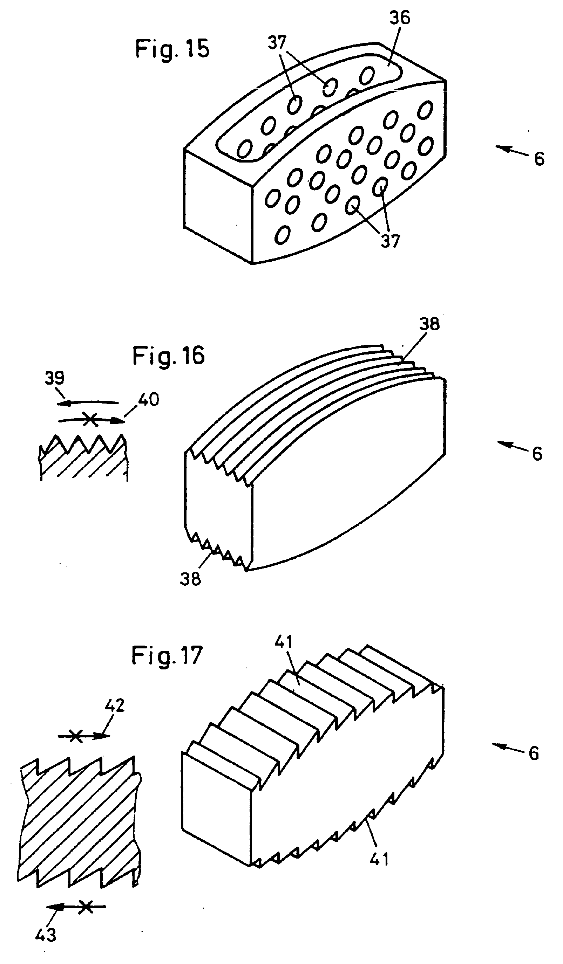

In Fig. 14 sind zwei flach im Zwischenwirbelraum 25 (Zeichnungsebene) liegende Implantate 6 dargestellt, die über ein Konnektor 34 anterior miteinander verbunden sind. Im linken Teil der Fig. 14 ist die Stellung vor der Rotation der Implantate 6 dargestellt, im rechten Teil der Fig. 14 nach erfolgter Rotation um 90° in die Konkavität der Deckplatten der angrenzenden Wirbelkörper.
Das posteriore Ende der Implantate 6 bleibt frei und ist nur anterior durch den Konnektor 34 verbunden, so dass (a) der Abstand zwischen dem linken und rechten Implantat 6 und deren Ausrichtung zueinander aufrechterhalten wird, (b) die Implantate 6 um ihre Längsachse 35 drehbar sind, und (c) die beiden Implantate 6 vor deren Implantation und/oder in situ mit dem Konnektor 34 koppelbar sind.

In Fig. 15 ist ein Implantat 6 mit einem Längsschnitt 35 zur Aufnahme von spongiösen Knochenmaterial oder osteokonduktives bzw. osteoinduktives Material und Querperforationen 36 der Wände für das Knochenwachstum dargestellt. Der Durchmesser der Perforationen 36 ist vorzugsweise derart konzipiert ist, dass (a) in die Längsöffnung 36 gepresste Spongiosa nicht seitlich austritt, und (b) die in der Spongiosa enthaltene Flüssigkeit beim Stopfen der Implantate 6 seitlich austreten und nach der Implantation wieder zurückdiffundieren kann, um ein postoperatives Schwellen der Spongiosa zu bewirken; und (c) Knochen durch die Perforationen 37 in das Implantat (6) hineinwachsen kann.

In Fig. 16 ist ein Implantat 6 dargestellt, dessen Kontaktflächen zwischen Implantat 6 und Knochen mit einer Längsstrukturierung 38 versehen ist. Die Längsstrukturierung 38 ist vorzugsweise derart gestaltet, dass das Rotieren des Implantates 6 in die Konkavität der Deckplatten nur in einer Richtung möglich ist, wie dies durch die Pfeile 39,40 angedeutet ist.

In Fig. 17 ist ein Implantat 6 dargestellt, dessen Kontaktflächen zwischen Implantat 6 und Knochen mit einer Querstrukturierung 41 versehen ist. Die Querstrukturierung 41 ist vorzugsweise derart gestaltet, dass die eine Kontaktfläche eine Translation in anteriorer und die andere eine Translation in posteriorer Richtung verhindert, wie dies durch die Pfeile 42,43 angedeutet ist. Die Verhinderung der Translation in anteriorer Richtung führt zu einer Entlastung des verbliebenen Annulus, der nach jüngster Forschung innerviert ist und damit auf anterioren Druck mit Schmerzsignalen reagieren könnte.

Diese Erfindung ist keinesfalls auf die als Beispiele gegebenen und in den Abbildungen dargestellten Modelle begrenzt - derartige Dilatatoren und das dazugehörende Werkzeug können in unterschiedlichen Formen und Grössen gefertigt werden, ohne dabei aus dem Rahmen der Definitionen, die in der Zusammenfassung im Anhang gegeben werden, zu fallen.

## Patentansprüche

1. implantat (6) für den Zwischenwirbelraum (25) mit
a) einem im wesentlichen quaderförmigen Körper (7) mit den Kantenlängen a,b,c;
b) einer vorderen axialen Endfläche (12) und einer hinteren axialen Endfläche (10), welche von der Längsachse (35) durchstossen werden;
c) zwei Seitenflächen (14,15) welche von einer Querachse (44) durchstossen werden;
d) einer Oberfläche (16) und einer Unterfläche (17) welche von einer Querachse (45) durchstossen werden; wobei
e) die vordere axiale Endfläche (12) und/oder die hinteren axialen Endfläche (10) im wesentlichen rechteckig ausgebildet sind;
f) die in Richtung der Querachse (44) gemessene Kantenlänge a des Körpers (7) und die in Richtung der Querachse (45) gemessene Kantenlänge b des Körpers (7) verschieden gross sind;
**dadurch gekennzeichnet, dass**,
g) das Implantat (6) zusätzlich eine Vorrichtung (8) zur Ergreifung durch ein Werkzeug (9) aufweist, welche derart beschaffen ist, dass sie die Ausübung einer Rotationskraft in beiden Richtungen um die Längsachse (35) auf das Implantat (6) ermöglicht;
h) das Implantat einen bezüglich der Längsachse (35) orthogonalen, teilweise abgerundeten Querschnitt aufweist;
i) das Implantat (6) am Längsschnitt des grössten Durchmessers (D2) ein linsenförmiges Profil aufweist; und
k) das vordere axiale Ende (12) des Körpers abgerundet oder keilförmig ausgebildet ist.

2. Implantat nach Anspruch 1, **dadurch gekennzeichnet, dass** eine durchgehende Öffnung (18) im Körper (7) vorgesehen ist, welche sich von der Oberfläche (16) bis zur Unterfläche (17) erstreckt und den Körper (7) hohl gestaltet.

3. Implantat nach Anspruch 2, **dadurch gekennzeichnet, dass** die durchgehende Öffnung (18) die Form einer länglichen Rille aufweist.

4. Implantat nach einem der Ansprüche 1 bis 3, **dadurch gekennzeichnet, dass** die Vorrichtung (8) mindestens aus einer in der Innenseite des Körpers (7) vorgesehenen Aussparung, in welche das, vorzugsweise schlüsselähnliche, Werkzeug (9) einführbar ist.

5. Implantat (6) nach einem der Ansprüche 1 bis 4, **dadurch gekennzeichnet, dass** das vordere axiale Ende (12) des Körpers (7) abgerundet oder keilförmig ausgebildet ist.

6. Implantat nach einem der Ansprüche 1 bis 3, **dadurch gekennzeichnet, dass** die Vorrichtung (8) so gestaltet ist, dass mindestens die Ausübung einer Axialkraft auf das Implantat (6) möglich ist.

7. Implantat nach einem der Ansprüche 1 bis 5, **dadurch gekennzeichnet, dass** die Vorrichtung (8) so gestaltet ist, dass mindestens die Ausübung einer Druckkraft auf das Implantat (6) möglich ist.

8. Implantat nach einem der Ansprüche 1 bis 5, **dadurch gekennzeichnet, dass** die Vorrichtung (8) so gestaltet ist, dass mindestens die Ausübung einer Zugkraft auf das Implantat (6) möglich ist.

9. Implantat nach einem der Ansprüche 1 bis 8, **dadurch gekennzeichnet, dass** es eine Vorrichtung (8) in der Form einer inneren Aussparung zur Anbringung eines Werkzeugs (9) aufweist, und dass in der Aussparung zwei Ansatzelemente (27) vorgesehen sind, die eine axiale Sperrung für das Werkzeug (9) aufweisen.

10. Implantat nach einem der Ansprüche 1 bis 9, **dadurch gekennzeichnet, dass** die Vorrichtung (8) so gestaltet ist, dass mindestens die Ausübung einer seitlichen Kraft auf das Implantat (6) möglich ist.

11. Implantat nach einem der Ansprüche 1 bis 10, **dadurch gekennzeichnet, dass** die Seiten (14 und 15), zwischen denen sich der kleinste Durchmesser (D1) erstreckt, grösstenteils parallel und flach sind.

12. Implantat nach Anspruch 10 oder 11, **dadurch gekennzeichnet, dass** das Implantat (6) am Längsschnitt des grössten Durchmessers (D2) ein abgekantetes, linsenförmiges Profil aufweist.

13. Implantat nach Anspruch 12, **dadurch gekennzeichnet, dass** die Seiten (15,16), die das abgekantete, linsenförmige Profil einschliessen, in Querrichtung zum Implantat (6) mindestens teilweise flach sind.

14. Implantat nach einem der Ansprüche 1 bis 13, **dadurch gekennzeichnet, dass** sowohl die vordere axiale Endfläche (12) als auch die hintere axiale Endfläche (10) abgerundet ist.

15. Implantat nach einem der Ansprüche 1 bis 14, **dadurch gekennzeichnet, dass** der Körper (7) eine oder mehrere Öffnungen (18) zur Füllung mit Pfropfmaterial (24) aufweist, wobei die Öffnungen derart angebracht sind, dass das Pfropfmaterial (24) bei der endgültigen Position des Implantats (6) die Wirbelkörper (1,2) berührt.

16. Implantat nach einem der Ansprüche 1 bis 15, **dadurch gekennzeichnet, dass** die durchgehende Öffnung (18) in Form einer länglichen Rille parallele Wände (19,20) aufweist.

17. Implantat nach einem der Ansprüche 1 bis 16, **dadurch gekennzeichnet, dass** es an zwei seiner Enden mit Vorrichtung (8) zur Ergreifung durch ein Werkzeug (9) versehen ist.

18. Implantat nach einem der Ansprüche 1 bis 17, **dadurch gekennzeichnet, dass** es aus Titan oder einer Titanlegierung besteht.

19. Implantat nach einem der Ansprüche 1 bis 18, **dadurch gekennzeichnet, dass** es einen rechteckigen Querschnitt mit einer einseitigen Abrundung (31) aufweist.

20. Implantat nach Anspruch 19, **dadurch gekennzeichnet, dass** der Radius der einseitigen Abrundung (31) derart bemessen ist, dass
a) die Differenz zwischen der grösseren Seite des rechteckigen Querschnittes und der Diagonalen über die abgerundete Kante kleiner als 3 mm, vorzugsweise 1-2 mm beträgt;
und
b) die Kontaktfläche zum Knochen durch die Abrundung (31) um weniger als die Hälfte, vorzugsweise weniger als ein Drittel reduziert ist.

21. Implantat nach einem der Ansprüche 1 bis 20, **dadurch gekennzeichnet, dass** es einen rechteckigem Querschnitt mit einer doppelseitigen Abrundung (32) über die Diagonale aufweist.

22. Implantat nach Anspruch 21, **dadurch gekennzeichnet, dass** die Radien der doppelseitigen Abrundungen (32) derart bemessen sind, dass
a) die Differenz zwischen der grösseren Seite des Querschnittes und der Diagonalen über die abgerundeten Kanten kleiner als 3 mm, vorzugsweise 0,5 - 1,0 mm beträgt und
b) die kleinere Fläche des Implantates um weniger als die Hälfte, vorzugsweise weniger als ein Viertel reduziert ist.

23. Implantat nach einem der Ansprüche 1 bis 22, **dadurch gekennzeichnet, dass** die Aussparung aus einem in der Innenseite des Körpers (7) vorgesehenen InnenSechskant besteht.

24. Implantat nach einem der Ansprüche 21 bis 23, **dadurch gekennzeichnet, dass** es einen rechteckigen Querschnitt aufweist, welcher derart beschaffen ist, dass nach der Rotation des Implantates in die Konkavität der Deckplatten der angrenzenden Wirbelkörper eine Distraktion des Zwischenwirbelraumes (25) zwischen 1 und 4 mm verbleibt.

25. Implantat nach Anspruch 24, **dadurch gekennzeichnet, dass** eine Distraktion des Zwischenwirbelraumes (25) zwischen 2 - 3 mm verbleibt.

26. Implantat nach einem der Ansprüche 21 bis 24, **dadurch gekennzeichnet, dass** das posteriore Ende des Implantats (6) derart beschaffen ist, dass ein linkes und ein rechtes Implantat anterior derart mit einem Konnektor (34) verbindbar sind, dass
(a) der Abstand zwischen dem linken und rechten Implantat (6) und deren Ausrichtung aufrechterhalten wird;
(b) die Implantate (6) um ihre Längsachse (35) drehbar sind; und
(c) die zwei Implantate (6) vor deren Implantation und/oder in situ mit dem Konnektor (34) koppelbar sind.

27. Implantat nach einem der Ansprüche 21 bis 24, **dadurch gekennzeichnet, dass** es derart beschaffen ist, dass es vor dessen Implantation oder in situ mit einem zweiten Implantat (6) verbindbar ist.

28. Implantat nach Anspruch 27, **dadurch gekennzeichnet, dass** die Verbindung derart beschaffen ist, dass eine Rotation der Implantate (6) um ihre Längsachse (35) möglich ist.

29. Implantat nach einem der Ansprüche 21 bis 28, **dadurch gekennzeichnet, dass** es an seiner Oberfläche beschichtet ist, vorzugsweise mit Hydroxylapatit oder Titanplasma.

30. Implantat nach einem der Ansprüche 15, 16 und einem der Ansprüche 21 - 29, **dadurch gekennzeichnet, dass** es perforierte Wände aufweist, wobei die Perforationen (37) vorzugsweise lochartig sind und der Durchmesser der Löcher derart konzipiert ist, dass
(a) in die Längsöffnung gepresste Spongiosa nicht seitlich austritt, und
(b) die in der Spongiosa enthaltene Flüssigkeit beim Stopfen der Implantate seitlich austreten und nach der Implantation wieder zurückdiffundieren kann, um ein postoperatives Schwellen der Spongiosa zu bewirken; und
(c) Knochen durch die Perforation in das Implantat hineinwachsen kann.

31. Implantat nach einem der Ansprüche 21 - 30, **dadurch gekennzeichnet, dass** die eine Kontaktfläche zwischen Implantat und Knochen eine Rasterung in der Längsrichtung des Implantates aufweist.

32. Implantat nach einem der Ansprüche 21 - 30, **dadurch gekennzeichnet, dass** beide Kontaktflächen zwischen Implantat und Knochen eine Rasterung in der Längsrichtung des Implantates aufweisen.

33. Implantat nach einem der Ansprüche 31 oder 32, **dadurch gekennzeichnet, dass** die Geometrie der Rasterung ein Rotieren des Implantates in eine Richtung zulässt und in die andere Richtung verhindert.

34. Implantat nach einem der Ansprüche 21 - 30, **dadurch gekennzeichnet, dass** die eine Kontaktfläche zwischen Implantat und Knochen eine Querrasterung aufweist.

35. Implantat nach einem der Ansprüche 21 - 30, **dadurch gekennzeichnet, dass** beide Kontaktflächen zwischen Implantat und Knochen eine Querrasterung aufweisen.

36. Implantat nach einem der Ansprüche 34 oder 35, **dadurch gekennzeichnet, dass** die Geometrie der Rasterung ein Verschieben des Implantates in anteriorer Richtung verhindert.

37. Implantat nach einem der Ansprüche 34 oder 35, **dadurch gekennzeichnet, dass** die Geometrie der Rasterung ein Verschieben des Implantates in posteriorer Richtung verhindert.

38. Implantat nach Anspruch 34, **dadurch gekennzeichnet, dass** die Geometrie der Rasterung der beiden Kontaktflächen zwischen Implantat und Knochen derart gestaltet ist, dass die eine Rasterung ein Verschieben des Implantates in anteriorer Richtung und die andere ein Verschieben in posteriorer Richtung verhindert.

39. Implantat nach einem der Ansprüche 31 oder 34, **dadurch gekennzeichnet, dass** es eine Längsrasterung der einen und eine Querrasterung der anderen Kontaktfläche aufweist.

40. Implantat nach einem der Ansprüche 34-37 oder 39, **dadurch gekennzeichnet, dass** die Querrasterung der einzelnen Kontaktflächen derart ausgelegt sind, dass ein Verschieben in posteriorer und anteriorer Richtung verhindert wird.

## Claims

1. Implant (6) for the inter-vertebral space (25) with
a) an essentially cuboid shaped body (7) with the edge lengths a, b, c;
b) an axial front surface (12) and an axial rear surface (10), penetrated by a longitudinal axis (35);
c) two lateral surfaces (14, 15) penetrated by a transversal axis (44);
d) an upper surface (16) and a lower surface (44) penetrated by a transversal axis (45); where
e) the axial front end surface (12) and/or the axial rear end surface (10) are essentially configured in a rectangular form;
f) the edge length a of the body (7) measured in the direction of the transversal axis (44) and the edge length b of the body (7) measured in the direction of the transversal axis (45) are of different length;
**characterized in that**
g) the implant (6) also presents a device (8) for grasping by a tool (9), which is shaped so as to allow exerting a rotational force in both directions around the longitudinal axis (35) on the implant (6),
h) the implant presents a cross section orthogonal to the longitudinal axis which is partially rounded off;
i) the implant (6) presents a lenticular profile in the longitudinal section of its largest diameter (D2); and
k) the axial front end (12) of the body is configured in a rounded off or wedge-shaped manner.

2. Implant according to claim 1, **characterized in that** a continuous opening (18) is provided in the body (7), which extends from the upper surface (16) to the lower surface (17) and shapes the body (7) in a hollow manner.

3. Implant according to claim 2, **characterized in that** the continuous opening (18) presents the form of a longitudinal groove.

4. Implant according to one of the claims from 1 to 3, claim 1, **characterized in that** the device (8) consists of at least one cavity provided inside the body (7), in which the preferably key-shaped tool (9) can be inserted.

5. Implant (6) according to one of the claims from 1 to 4, **characterized in that** the axial front end (12) of the body (7) is configured in a rounded off or wedge-shaped form.

6. Implant according to one of the claims from 1 to 3, **characterized in that** the device (8) is configured so that it allows exerting at least an axial force on the implant (6).

7. Implant according to one of the claims from 1 to 5, **characterized in that** the device (8) is configured so that it allows exerting at least a pressing force on the implant (6).

8. Implant according to one of the claims from 1 to 5, **characterized in that** the device (8) is configured so that it allows exerting at least a pulling force on the implant (6).

9. Implant according to one of the claims from 1 to 8, **characterized in that** it presents a device (8) in the form of an internal cavity for applying a tool (9), and that two attaching elements (27) with an axial locking action for the tool (9) are provided in the cavity.

10. Implant according to one of the claims from 1 to 9, **characterized in that** the device (8) is configured so as to allow exerting at least a lateral force on the implant.

11. Implant according to one of the claims from 1 to 10, **characterized in that** the sides (14 and 15) with the smallest diameter (D1) extending between are mostly parallel and flat.

12. Implant according to claim 10 or 11, **characterized in that** the implant presents a chamfered, lenticular profile in the longitudinal section of its largest diameter (D2).

13. Implant according to claim 12, **characterized in that** the sides (15, 16) including the chamfered, lenticular profile are at least partially flat in a direction transversal to the implant.

14. Implant according to one of the claims from 1 to 13, **characterized in that** both the axial front end surface (12) as well as the axial rear end surface (10) are rounded off.

15. Implant according to one of the claims from 1 to 14, **characterized in that** the body (7) presents one or several openings (18) to be filled with plugging material (24), where the openings are arranged so that the plugging material (24) touches the vertebral bodies (1,2) in the final position of the implant.

16. Implant according to one of the claims from 1 to 15, **characterized in that** the continuous opening (18) presents parallel walls (19, 20) in the form of an elongated groove.

17. Implant according to one of the claims from 1 to 16, **characterized in that** it is fitted at two of its extremities with a device (8) suitable for grasping by a tool (9).

18. Implant according to one of the claims from 1 to 17, **characterized in that** it consists of titanium or a titanium alloy.

19. Implant according to one of the claims from 1 to 18, **characterized in that** it presents a rectangular cross section with a single sided rounding (31).

20. Implant according to claim 19, **characterized in that** the radius of the single-sided rounding (31) is sized so that
a) the difference between the larger side of the rectangular cross section and the diagonal line across the rounded off edge is smaller than 3 mm, preferably 1 - 2 mm, and
b) the contact surface facing the bone is reduced by the rounding (31) by less than half, preferably less than one third.

21. Implant according to one of the claims from 1 to 20, **characterized in that** it presents a rectangular cross section with a double-sided rounding (32) across its diagonal line.

22. Implant according to claim 21, **characterized in that** the radii of the double sided roundings (32) are sized so that
a) the difference between the larger side of the cross section and the diagonal line across the rounded off edges is smaller than 3 mm, preferably 0.5 - 1.0 mm, and
b) the smaller surface of the implant is reduced by less than half, preferably by less than one quarter.

23. Implant according to one of the claims from 1 to 22, **characterized in that** the cavity consists of a hexagonal recess provided on the inner side of the body (7).

24. Implant according to one of the claims from 21 to 23, **characterized in that** it presents a rectangular cross section configured so that after rotating the implant in the cavity of the cover plates of adjacent vertebral bodies, a distraction of the inter-vertebral space (25) between 1 and 4 mm remains.

25. Implant according to claim 24, **characterized in that** a distraction of the inter-vertebral space (25) of 2 - 3 mm remains.

26. Implant (6) according to one of the claims from 21 to 24, **characterized in that** the posterior end of the implant (6) is configured so that a left and a right implant can be joined anteriorly by a connector (34) so that
a) the distance between the left and the right implant (6) and their alignment is maintained; and
b) the implants (6) can be rotated around their longitudinal axis (35); and
c) the two implants (6) can be connected, before their implantation and/or in situ, with the connector (34).

27. Implant according to one of the claims from 21 to 24, **characterized in that** it is configured so that it can be connected with a second implant (6) before its implantation or in situ.

28. Implant according to claim 27, **characterized in that** the connection is configured so that a rotation of the implants (6) around their longitudinal axis (35) is possible.

29. Implant according to one of the claims from 21 to 28, **characterized in that** it is coated on its surface, preferably with a hydroxy-apatite or titanium plasma.

30. Implant (6) according to one of the claims 15, 16 and one of the claims from 21 to 29, **characterized in that** it presents perforated walls, where the perforations (37) are preferably like holes and the diameter of the holes is conceived so that
a) spongiosa pressed in the longitudinal opening cannot exit laterally,
b) during the stuffing of the implants the liquid contained in the spongiosa can exit laterally and diffuse backward after the implantation, to create a postoperative swelling of the spongiosa; and
c) bone can grow into the implant across the perforations.

31. Implant according to one of the claims from 21 to 30, **characterized in that** the one contact surface between the implant and the bones presents a lenticulation in the longitudinal direction of the implant.

32. Implant according to one of the claims from 21 to 30, **characterized in that** both contact surfaces between the implant and the bones present a lenticulation in the longitudinal direction of the implant.

33. Implant according to one of the claims 31 or 32, **characterized in that** the geometry of the lenticulation allows a rotation of the implant in one direction and prevents a rotation in the other direction.

34. Implant according to one of the claims from 21 to 30, **characterized in that** the one contact surface between the implant and the bones presents a transversal lenticulation.

35. Implant according to one of the claims from 2 to 30, **characterized in that** both contact surfaces present a transversal lenticulation between the implant and the bones.

36. Implant according to one of the claims 34 or 35, **characterized in that** the geometry of the lenticulation prevents a shifting of the implant in the anterior direction.

37. Implant according to one of the claims 34 or 35, **characterized in that** the geometry of the lenticulation prevents prevents a shifting of the implant in the posterior direction.

38. Implant according to claim 34, **characterized in that** the geometry of the lenticulation of the two contact surfaces between implant and bone is configured so that one lenticulation prevents a shifting of the one implant in the anterior direction, and the other a shifting in the posterior direction.

39. Implant according to one of the claims 31 or 34, **characterized in that** it presents a longitudinal lenticulation of the one contact surface, and a transversal lenticulation of the other contact surface.

40. Implant according to one of the claims 34 - 37 or 39, **characterized in that** the transversal lenticulation of the individual contact surfaces is arranged so that a shifting in posterior and anterior direction is prevented.

## Revendications

1. Implant (6) pour l'espace intervertébral (25), comprenant
a) un corps (7) essentiellement parallélépipédique avec les longueurs de côté a, b, c ;
b) une surface d'extrémité axiale avant (12) et une surface d'extrémité axiale arrière (10), lesquelles sont traversées par l'axe longitudinal (35);
c) deux faces latérales (14, 15), lesquelles sont traversées par l'axe transversal (44);
d) une face supérieure (16) et une face inférieure (17), lesquelles sont traversées par un axe transversal (45) ; dans lequel
e) la surface d'extrémité axiale avant (12) et/ou la surface d'extrémité axiale arrière (10) sont essentiellement rectangulaires ;
f) la longueur de côté a du corps (7) mesurée dans le sens de l'axe transversal (44) et la longueur de côté b du corps (7) mesurée dans le sens de l'axe transversal (45) sont différentes;
**caractérisé en ce que**
g) l'implant (6) présente en outre un dispositif (8) pouvant être saisi par un outil (9), qui est conçu de telle manière qu'il permet d'exercer une force de rotation sur l'implant (6) dans les deux sens autour de l'axe longitudinal (35) ;
h) l'implant présente une coupe droite partiellement arrondie, orthogonale à l'axe longitudinal (35) ;
i) l'implant (6) présente un profil lenticulaire au niveau de la coupe longitudinale du diamètre le plus grand (D2) ; et
k) l'extrémité axiale avant (12) du corps est arrondie ou en forme de coin.

2. Implant selon la revendication 1, **caractérisé en ce qu'**une ouverture continue (18) est prévue dans le corps (7), laquelle s'étend de la face supérieure (16) jusqu'à la face inférieure (17) et rend le corps (7) creux.

3. Implant selon la revendication 2, **caractérisé en ce que** l'ouverture continue (18) a la forme d'une gorge longitudinale.

4. Implant selon l'une quelconque des revendications 1 à 3, **caractérisé en ce que** le dispositif (8) est formé par au moins un évidement réalisé dans la face interne du corps (7), dans lequel l'outil (9), de préférence analogue à une clé, peut être inséré.

5. Implant (6) selon l'une quelconque des revendications 1 à 4, **caractérisé en ce que** l'extrémité axiale avant (12) du corps (7) est arrondie ou en forme de coin.

6. Implant selon l'une quelconque des revendications 1 à 3, **caractérisé en ce que** le dispositif (8) est conçu de telle manière qu'au moins l'application d'une force axiale sur l'implant (6) est possible.

7. Implant selon l'une quelconque des revendications 1 à 5, **caractérisé en ce que** le dispositif (8) est conçu de telle manière qu'au moins l'application d'une force de pression sur l'implant (6) est possible.

8. Implant selon l'une quelconque des revendications 1 à 5, **caractérisé en ce que** le dispositif (8) est conçu de telle manière qu'au moins l'application d'une force de traction sur l'implant (6) est possible.

9. Implant selon l'une quelconque des revendications 1 à 8, **caractérisé en ce qu'**il présente un dispositif (8) sous la forme d'un évidement interne permettant d'y introduire un outil (9), et **en ce que** deux éléments de raccordement (27), qui présentent un arrêt axial pour l'outil (9), sont prévus dans l'évidement.

10. Implant selon l'une quelconque des revendications 1 à 9, **caractérisé en ce que** le dispositif (8) est conçu de telle manière qu'au moins l'application d'une force latérale sur l'implant (6) est possible.

11. Implant selon l'une quelconque des revendications 1 à 10, **caractérisé en ce que** les faces (14 et 15), entre lesquelles s'étend le diamètre le plus petit (D1), sont essentiellement parallèles et planes.

12. Implant selon la revendication 10 ou 11, **caractérisé en ce que** l'implant (6) présente, au niveau de la coupe longitudinale du diamètre le plus grand (D2), un profil lenticulaire chanfreiné.

13. Implant selon la revendication 12, **caractérisé en ce que** les faces (15, 16), qui renferment le profil lenticulaire chanfreiné, sont au moins partiellement planes dans le sens transversal à l'implant (6).

14. Implant selon l'une quelconque des revendications 1 à 13, **caractérisé en ce qu'**aussi bien la surface d'extrémité axiale avant (12) que la surface d'extrémité axiale arrière (10) sont arrondies.

15. Implant selon l'une quelconque des revendications 1 à 14, **caractérisé en ce que** le corps (7) présente une ou plusieurs ouvertures (18) pour le remplissage avec un matériau de comblement (24), dans lequel les ouvertures sont placées de telle manière que le matériau de comblement (24) touche les corps vertébraux (1, 2) lorsque l'implant (6) se trouve dans sa position définitive.

16. Implant selon l'une quelconque des revendications 1 à 15, **caractérisé en ce que** l'ouverture continue (18) en forme de gorge longitudinale présente des parois parallèles (19, 20).

17. Implant selon l'une quelconque des revendications 1 à 16, **caractérisé en ce qu'**il est pourvu au niveau de deux de ses extrémités, d'un dispositif (8) pouvant être saisi par un outil (9).

18. Implant selon l'une quelconque des revendications 1 à 17, **caractérisé en ce qu'**il est fait de titane ou d'un alliage de titane.

19. Implant selon l'une quelconque des revendications 1 à 18, **caractérisé en ce qu'**il présente une coupe droite rectangulaire avec un arrondi unilatéral (31).

20. Implant selon la revendication 19, **caractérisé en ce que** le rayon de l'arrondi unilatéral (31) est dimensionné de telle manière que
a) la différence entre le côté le plus grand de la coupe droite rectangulaire et la diagonale passant par le bord arrondi est inférieure à 3 mm, en étant de préférence de 1 à 2 mm ;
et
b) la surface de contact avec l'os est réduite par l'arrondi (31) de moins de la moitié, de préférence de moins du tiers.

21. Implant selon l'une quelconque des revendications 1 à 20, **caractérisé en ce qu'**il présente une coupe droite rectangulaire avec un arrondi bilatéral (32) sur la diagonale.

22. Implant selon la revendication 21, **caractérisé en ce que** les rayons des arrondis bilatéraux (32) sont dimensionnés de telle manière que
a) la différence entre le côté le plus grand de la coupe droite et la diagonale passant par les bords arrondis est inférieure à 3 mm, en étant de préférence de 0,5 à 1,0 mm ;
et
b) la surface la plus petite de l'implant est réduite de moins de la moitié, de préférence de moins du quart.

23. Implant selon l'une quelconque des revendications 1 à 22, **caractérisé en ce que** l'évidement est constitué par un creux hexagonal prévu dans la face interne du corps (7).

24. Implant selon l'une quelconque des revendications 21 à 23, **caractérisé en ce qu'**il présente une coupe droite rectangulaire, laquelle est conçue de telle manière qu'une distraction de l'espace intervertébral (25) comprise entre 1 et 4 mm persiste après la rotation de l'implant dans la concavité des plaques de recouvrement des corps vertébraux adjacents.

25. Implant selon la revendication 24, **caractérisé en ce qu'**une distraction de l'espace intervertébral (25) comprise entre 2 et 3 mm persiste.

26. Implant selon l'une quelconque des revendications 21 à 24, **caractérisé en ce que** l'extrémité postérieure de l'implant (6) est conçue de telle manière qu'un implant gauche et un implant droit peuvent être reliés avec un connecteur (34) par voie antérieure, si bien que
(a) la distance entre les implants gauche et droits (6) et leur orientation sont conservés ;
(b) les implants (6) peuvent tourner autour de leur axe longitudinal (35) ;
et
(c) les deux implants (6) peuvent être couplés, avant leur implantation et/ou *in situ,* à l'aide du connecteur (34).

27. Implant selon l'une quelconque des revendications 21 à 24, **caractérisé en ce qu'**il est conçu de telle manière qu'il peut être relié, avant son implantation ou *in situ,* à un deuxième implant (6).

28. Implant selon la revendication 27, **caractérisé en ce que** la liaison est conçue de telle manière qu'une rotation des implants (6) autour de leur axe longitudinal (35) est possible.

29. Implant selon l'une quelconque des revendications 21 à 28, **caractérisé en ce qu'**il est revêtu en surface, de préférence avec de l'hydroxyapatite ou un plasma de titane.

30. Implant selon l'une quelconque des revendications 15, 16 et l'une quelconque des revendications 21 à 29, **caractérisé en ce qu'**il présente des parois perforées, dans lequel les perforations (37) sont de préférence en forme de trous et le diamètre des trous est défini de telle manière que
(a) de l'os spongieux injecté dans l'ouverture longitudinale ne ressort pas sur les côtés, et
(b) le liquide contenu dans l'os spongieux peut ressortir sur les côtés lorsque les implants sont bouchés et se rediffuser après l'implantation pour entraîner un gonflement postopératoire de l'os spongieux ; et
(c) l'os peut croître à travers la perforation dans l'implant.

31. Implant selon l'une quelconque des revendications 21 à 30, **caractérisé en ce qu'**une surface de contact entre l'implant et l'os présente une trame dans le sens longitudinal de l'implant.

32. Implant selon l'une quelconque des revendications 21 à 30, **caractérisé en ce que** les deux surfaces de contact entre l'implant et l'os présentent une trame dans le sens longitudinal de l'implant.

33. Implant selon l'une quelconque des revendications 31 ou 32, **caractérisé en ce que** la géométrie de la trame permet une rotation de l'implant dans un sens et l'empêche dans l'autre sens.

34. Implant selon l'une quelconque des revendications 21 à 30, **caractérisé en ce qu'**une surface de contact entre l'implant et l'os présente une trame transversale.

35. Implant selon l'une quelconque des revendications 21 à 30, **caractérisé en ce que** les deux surfaces de contact entre l'implant et l'os présentent une trame transversale.

36. Implant selon l'une quelconque des revendications 34 ou 35, **caractérisé en ce que** la géométrie de la trame empêche un déplacement de l'implant dans la direction antérieure.

37. Implant selon l'une quelconque des revendications 34 ou 35, **caractérisé en ce que** la géométrie de la trame empêche un déplacement de l'implant dans la direction postérieure.

38. Implant selon la revendication 34, **caractérisé en ce que** la géométrie de la trame des deux surfaces de contact entre l'implant est l'os est conçue de telle manière qu'une trame empêche un déplacement de l'implant dans la direction antérieure et l'autre empêche un déplacement dans la direction postérieure.

39. Implant selon l'une quelconque des revendications 31 ou 34, **caractérisé en ce qu'**il présente une trame longitudinale sur l'une des surfaces de contact et une trame transversale sur l'autre surface de contact.

40. Implant selon l'une quelconque des revendications 34 à 37 ou 39, **caractérisé en ce que** la trame transversale des surfaces de contact individuelles est conçue de telle manière qu'un déplacement dans la direction postérieure et antérieure est empêché.
